(19) Europäisches Patentamt
European Patent Office
Office européen des brevets

(11) Veröffentlichungsnummer: **0 000 940**
**B1**

(12) **EUROPÄISCHE PATENTSCHRIFT**

(21) Anmeldenummer: **78100725.7**

(22) Anmeldetag: **23.08.78**

(51) Int. Cl.³: **C 07 D 249/08,** C 07 D 233/60, C 07 D 249/04, C 07 D 231/12, C 07 D 405/06, C 07 D 409/06, C 07 D 401/06, A 01 N 43/50, A 01 N 43/56, A 01 N 43/64

(54) **Gamma-Azolylalkohole, und ihre Verwendung zur Regulierung des Pflanzenwachstums**

(30) Priorität: **27.08.77 DE 2738725**

(43) Veröffentlichungstag der Anmeldung:
**07.03.79 Patentblatt 7905**

(45) Bekanntmachung des Hinweises auf die Patenterteilung:
**06.08.80 Patentblatt 8016**

(84) Benannte Vertragsstaaten:
**BE CH DE FR GB LU NL SE**

(56) Entgegenhaltung:
**NL - A - 7 708 444**

(73) Patentinhaber: **BASF Aktiengesellschaft**
**Carl - Bosch - Strasse 38**
**D - 6700 Ludwigshafen (DE)**

(72) Erfinder: **Rentzea, Costin, Dr.**
**Neuenheimer Landstrasse 27**
**D - 6900 Heidelberg (DE)**
**Jung, Johann, Dr.**
**Hardenburgstrasse 19**
**D - 6703 Limburgerhof (DE)**
**Sauter, Hubert, Dr.**
**Goethestrasse 23**
**D - 6700 Ludwigshafen (DE)**
**Heilen, Gerd, Dr.**
**Schifferstadter Strasse 1b**
**D - 6720 Speyer (DE)**
**Zeeh, Bernd, Dr.**
**Thorwaldsenstrasse 5**
**D - 6700 Ludwigshafen (DE)**

EP 0 000 940 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Gamma-Azolylalkohole, und ihre Verwendung zur Regulierung des Pflanzenwachstums

Die vorliegende Erfindung betrifft neue wertvolle γ-Azolylalkohole mit guter biologischer Wirkung, Mittel zur Regulierung des Pflanzenwachstums, die diese Verbindungen enthalten, und Verfahren zur Regulierung des Pflanzenwachstums mit diesen Verbindungen.

Es ist bekannt, daß 2-Chloräthyl-trimethylammoniumchlorid (Chlorcholinchlorid, CCC) (J. Biol. Chem., *235,* 475 (1960) zur Beeinflussung des Pflanzenwachstums zu verwenden.

Pflanzenwachstumsregulatoren können z.B. eine Reduzierung des Längenwachstums verursachen, eine Keim- oder Blühinduktion auslösen, die Frostresistenz erhöhen, oder die Ausbildung von Seitentrieben fördern bzw. hemmen.

Von großem wirtschaftlichen Interesse ist besonders die Verhinderung des "Lagerns" von Getreide vor der Ernte durch Wachstumsregulatoren.

Bei der Anwendung der bekannten Handelsprodukte als Mittel zur Regulierung des Pflanzenwachstums, z.B. auf Getreidearten, bei der ein gedrungener Wuchs zur Unterbindung des Lagerns erreicht werden soll, ist die Wirkung oft nicht ausreichend.

Es wurde gefunden, daß γ-Azolylalkohole der allgemeinen Formel

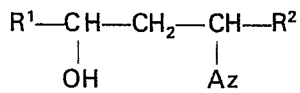

I

in der $R^1$ und $R^2$ gleich oder verschieden sind und Alkyl mit bis zu 5 C-Atomen, Cycloalkyl, Furanyl, Thiophenyl, Pyridyl, Naphthyl oder Phenyl bedeuten, wobei der Phenylrest durch Halogen, Alkyl, Alkoxy, Alkenyloxy, Hydroxy, Nitro, Trifluormethyl substituiert sein kann und $R^1$ auch noch Biphenyl sein kann und Az einen Imidazolyl-, Pyrazolyl-, 1,2,4-Triazolyl- oder einen 1,2,3-Triazolyrest bedeutet, eine sehr gute pflanzenwachstumsregulierende Wirksamkeit zeigen.

Die erfindungsgemäßen γ-Azolylalkohole eignen sich sehr gut bei zahlreichen Pflanzen zur Regulierung des Wachstums, was sich insbesondere in einer Reduzierung des Längenwachstums äußert. Die Wirkung ist besser als bei bekannten Wachstumsregulatoren.

Die Wirkung zeigt sich vor allem bei Getreide, z.B. Weizen, Roggen, Gerste, Reis und Hafer, aber auch bei Dikotylen (z.B. Sonnenblumen, Tomaten, Reben, Baumwolle, Raps) und verschiedenen Zierpflanzen, wie Poinsettien und Hibiskus. Die behandelten Pflanzen weisen demgemäß einen niedrigeren Wuchs auf; außerdem ist eine dunklere Blattfärbung zu beobachten.

Die erfindungsgemäßen γ-Azolylalkohole können den Kulturpflanzen sowohl über den Boden, d.h. durch die Wurzel, als auch durch Spritzung über das Blatt zugeführt werden. Infolge der hohen Pflanzenverträglichkeit kann die Aufwandmenge stark variiert werden; im allgemeinen sind aber Gaben von 0,25 bis 12 kg/ha als ausreichend zu betrachten.

$R^1$ bedeutet beispielsweise Methyl, Isopropyl, n-Butyl, sek.-Butyl, iso-Butyl, tert.-Butyl, Furfuryl, Thiophenyl, Naphthyl, Biphenylyl, Phenyl, p-Tolyl, m- und p-Methoxy-phenyl, p-Fluorphenyl, p-Chlorphenyl oder 2,4-Dichlor-phenyl.

$R^2$ bedeutet beispielsweise tert.-Butyl, Furfuryl, 2-Methyl-furfuryl, Thiophenyl, Pyridyl, Naphthyl, Phenyl, p-Tolyl, p-Isopropyl-phenyl, o- und p-Methoxy-phenyl, 2,3-Dimethoxy-phenyl, o-Hydroxyphenyl, p-Chlorphenyl, m- und p-Bromphenyl, 2,4-Dichlorphenyl, 2,4-Dibromphenyl oder p-Nitrophenyl.

Die erfindungsgemäßen Verbindungen der Formel I sind neu und können in folgender Weise hergestellt werden. Man erhält sie beispielsweise, indem man ß-Azolylketone der Formel

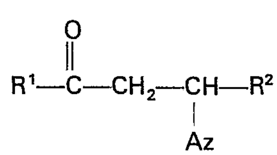

II

in welcher $R^1$, $R^2$ und Az die oben angegebene Bedeutung haben, reduziert. Die Reduktion kann zum Beispiel durchgeführt werden:

a) mit komplexen Hydriden, wie Natriumborhydrid in Gegenwart eines polaren Lösungsmittels, wie zum Beispiel Methanol oder mit Lithiumalanat in Tetrahydrofuran, bei Temperaturen zwischen 0°C und 30°C und anschließende Hydrolyse, z.B. mit wäßrigen Basen oder Säuren, oder

b) mit Wasserstoff in Gegenwart eines Katalysators, wie Platin oder Raney-Nickel und in Gegenwart eines polaren Lösungsmittels, wie Methanol, Äthanol, Isopropanol, bei einer Temperatur zwischen 40 und 60°C und einem Druck zwischen 1 bis 100 bar, und

c) mit Aluminiumisopropylat in Gegenwart eines inerten Lösungsmittels bei einer Temperatur zwischen 40 und 120°C und anschließender Hydrolyse, z.B. mit wäßriger Salzsäure.

Die so erhaltenen Verbindungen der Formel (I) werden nach den üblichen Methoden isoliert und gegebenenfalls gereinigt.

Die Ausgangsstoffe der Formel (II) können wie folgt hergestellt werden.

Man erhält die β-(1,2,4-Triazol-(1))-yl-, β-Imidazolyl- und β-Pyrazolyl-ketone der Formel (II), wenn man entsprechend substituierte α,β-ungesättigte Ketone mit den gewünschten Azolen in Gegenwart von Lösungs- oder Verdünnungsmittel bei Temperaturen zwischen 20 und 80°C umsetzt oder wenn man anstatt von α,β-ungesättigten Ketonen die entsprechenden Aldehyde und Ketone in Gegenwart von Azolen und gegebenenfalls in Gegenwart eines basischen Katalysators und gegebenenfalls in Gegenwart eines Verdünnungsmittels umsetzt.

Vorschrift A

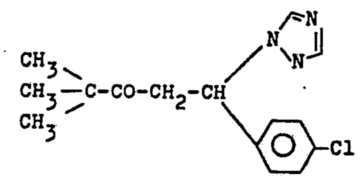

Eine Lösung von 11 g (0,11 Mol) 3,3-Dimethylbutan-2-on und 14 g (0,1 Mol) p-Chlorbenzaldehyd in 120 ml Äthanol werden mit 10 ml 20 prozentiger wäßriger Natronlauge versetzt und 2 Stunden bei 50°C gerührt. Nach Zugabe von 13,8 g (0,2 Mol) 1,2,4-Triazol wird das Gemisch weitere 8 Stunden bei 50°C nachgerührt. Danach wird das Lösungsmittel abdestilliert und der verbleibende Rückstand zwischen 300 ml Wasser und 300 ml Äther verteilt.

Die ätherische Lösung wird über $CaSO_4$ getrocknet, abfiltriert und mit trockenem Chlorwasserstoff bei 5 bis 10° begast.

Man erhält 24,3 g (74% d. Th.) Hydrochlorid des 1-[1,2,4-Triazolyl-(1)]-1-(4'-chlor-phenyl)-4,4-dimethyl-pentan-3-on als weiße Kristalle vom Schmelzpunkt 139—141°C.

Folgende Ketone können beispielsweise als Ausgangsprodukte verwendet werden.

TABELLE 1

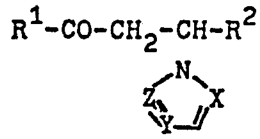

| Nr. | $R^1$ | $R^2$ | X | Y | Z | Fp °C | Fp (Hydrochlorid) °C |
|---|---|---|---|---|---|---|---|
| 1 | $(CH_3)_3C-$ | 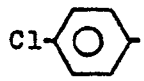 | N | N | CH | | 139–141 |
| 2 | $CH_3-$ | $(CH_3)_3C-$ | N | N | CH | | |
| 3 | $(CH_3)_2CH-$ | 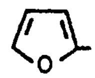 | N | N | CH | 53–55 | |
| 4 | $(CH_3)_2CH-$ | 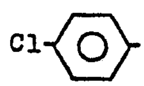 | N | N | CH | | 118–120 |
| 5 | sek.-$C_4H_9-$ | 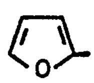 | N | N | CH | 41–43 | |
| 6 | iso-$C_4H_9-$ | 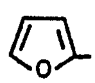 | N | N | CH | | 163–166 |
| 7 | $(CH_3)_3C-$ | 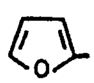 | N | N | CH | 69–71 | |
| 8 | $(CH_3)_3C-$ | 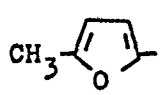 | N | N | CH | 49–51 | |
| 9 | $(CH_3)_3C-$ | 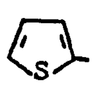 | N | N | CH | | 118–120 |
| 10 | sek.-$C_4H_9-$ | 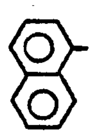 | N | N | CH | Harz | |

TABELLE 1

| Nr. | $R^1$ | $R^2$ | X | Y | Z | Fp °C | Fp (Hydrochlorid) °C |
|---|---|---|---|---|---|---|---|
| 11 | iso-$C_4H_9$– | 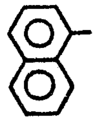 | N | N | CH | | 167–169 |
| 12 | $(CH_3)_3C$– | 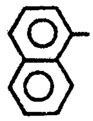 | N | N | CH | | 162–164 |
| 13 | $(CH_3)_3C$– | 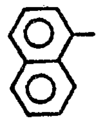 | N | CH | CH | 68–69 | |
| 14 | 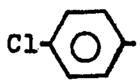 | $(CH_3)_3C$– | CH | N | N | 130–132 | |
| 15 | $(CH_3)_3C$– | 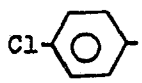 | CH | N | CH | 89–91 | |
| 16 | $(CH_3)_3C$– | 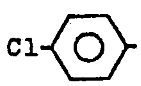 | N | CH | CH | 65–67 | |
| 17 | $(CH_3)_3C$– | 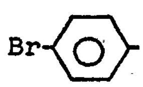 | N | N | CH | | 142–145 |
| 18 | $(CH_3)_3C$– | 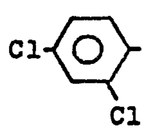 | N | N | CH | | 154–156 |
| 19 | $(CH_3)_3C$– | 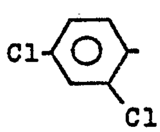 | CH | N | CH | 75–77 | |
| 20 | $(CH_3)_3C$– | 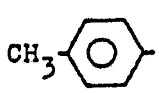 | N | N | CH | | 153–155 |

TABELLE 1

| Nr. | R[1] | R[2] | X | Y | Z | Fp °C | Fp (Hydrochlorid) °C |
|---|---|---|---|---|---|---|---|
| 21 | $(CH_3)_3C-$ | 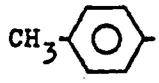 | CH | N | CH | 98–100 | |
| 22 | $(CH_3)_3C-$ | 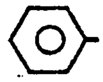 | N | N | CH | | 141–143 |
| 23 | $(CH_3)_3C-$ | 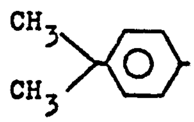 | N | N | CH | | 155–157 |
| 24 | $(CH_3)_3C-$ | 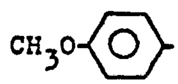 | N | N | CH | | 158–161 |
| 25 | $(CH_3)_3C-$ | 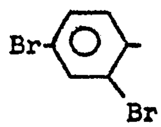 | N | N | CH | 104–106 | |
| 26 | $(CH_3)_3C-$ | 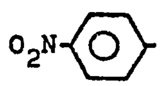 | N | N | CH | | 164–167 |
| 27 | 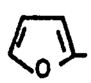 | 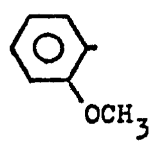 | N | N | CH | 106 | |
| 28 | 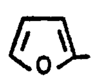 | 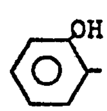 | N | N | CH | 142–144 | |
| 29 | 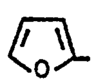 | 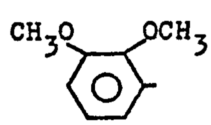 | N | N | CH | 101–102 | |
| 30 | 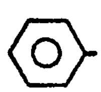 | 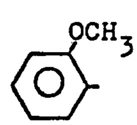 | N | N | CH | 95–97 | |

TABELLE 1

| Nr. | $R^1$ | $R^2$ | X | Y | Z | Fp °C | Fp (Hydrochlorid) °C |
|---|---|---|---|---|---|---|---|
| 31 | 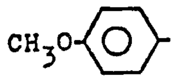 | 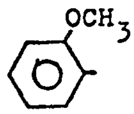 | N | N | CH | 134–135 | |
| 32 | 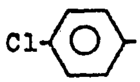 | 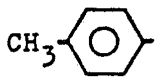 | N | N | CH | 81–82 | |
| 33 | 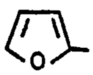 | 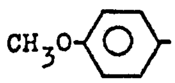 | N | N | CH | 126–128 | |
| 34 | 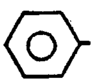 | 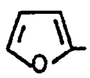 | N | N | CH | 126 | |
| 35 | 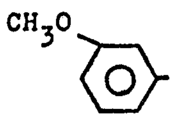 | 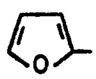 | N | N | CH | 130–133 | |
| 36 | 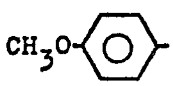 | 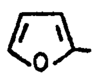 | N | N | CH | 112–114 | |
| 37 | $(CH_3)_3C-$ | 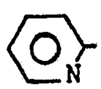 | N | N | CH | 76–79 | |
| 38 | 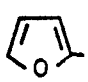 | $(CH_3)_3C-$ | N | N | CH | 104–106 | |
| 39 | 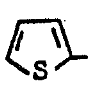 | $(CH_3)_3C-$ | N | N | CH | 125–127 | |
| 40 | 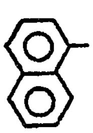 | $(CH_3)_3C-$ | N | N | CH | 136–138 | |

TABELLE 1

| Nr. | $R^1$ | $R^2$ | X | Y | Z | Fp °C | Fp (Hydrochlorid) °C |
|---|---|---|---|---|---|---|---|
| 41 | 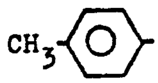 | $(CH_3)_3C-$ | N | N | CH | 128–130 | |
| 42 | 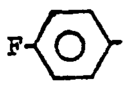 | $(CH_3)_3C-$ | N | N | CH | 100–102 | |
| 43 | 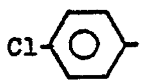 | $(CH_3)_3C-$ | N | N | CH | 133–115 | |
| 44 | 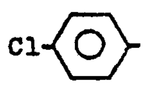 | $(CH_3)_3C-$ | CH | N | CH | 121–124 | |
| 45 | 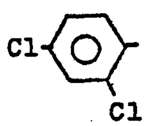 | $(CH_3)_3C-$ | N | N | CH | 70–72 | |
| 46 | 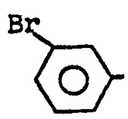 | $(CH_3)_3C-$ | N | N | CH | 114–116 | |
| 47 | 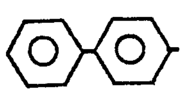 | $(CH_3)_3C-$ | N | N | CH | 127–128 | |
| 48 | $(CH_3)_3C-$ | $(CH_3)_3C-$ | N | N | CH | 98–99 | |
| 49 | 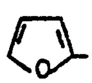 | 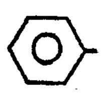 | N | N | CH | 106–108 | |
| 50 | 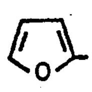 | 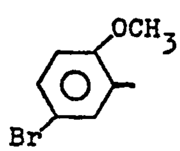 | N | N | CH | 146–148 | |
| 51 | $(CH_3)_3C-$ | 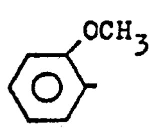 | N | N | CH | 92–95 | |

TABELLE 1

| Nr. | $R^1$ | $R^2$ | X | Y | Z | Fp °C | Fp (Hydrochlorid) °C |
|---|---|---|---|---|---|---|---|
| 52 | 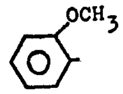 | 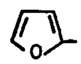 | N | N | CH | | |
| 53 | 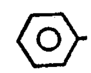 | 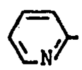 | N | N | CH | | |
| 54 | 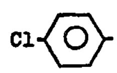 | 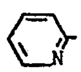 | N | N | CH | 78–81 | |
| 55 | 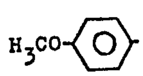 | 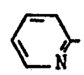 | N | N | CH | 108–110 | |

Das folgende Beispiel betrifft die Herstellung der erfindungsgemäßen γ-Azolylalkohole.

BEISPIEL 1

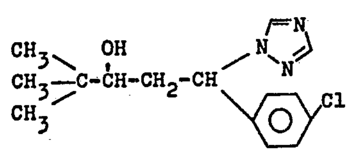

1a)

In eine Lösung von 29,1 g 1-[1,2,4-Triazolyl-(1)]-1-(4′-chlorphenyl)-4,4-dimethyl-pentan-3-on in 300 ml Methanol werden unter Rühren und Eiskühlung bei 3 bis 8°C portionsweise 5,9 g Natriumborohydrid eingetragen. Nach 8-stündigem Rühren bei Raumtemperatur wird das Gemisch eingeengt. Der Rückstand wird mit 150 ml 4 prozentiger Natronlauge 1 Stunde gerührt und mit 250 ml Methylenchlorid extrahiert. Die organische Schicht wird über $Na_2SO_4$ getrocknet und im Vakuum eingeengt. Der verbleibende feste, farblose Rückstand wird mit 40 ml Petroläther gewaschen und abgesaugt.

Man erhält 26,1 g (89% d. Th.) 1-[1,2,4-Triazolyl-(1)]-1-(4′-chlor-phenyl)-4,4-dimethyl-pentan-3-ol vom Schmelzpunkt 104—106°C.

1b)

29,1 g 1-[1,2,4-Triazolyl-(1)]-1-(4′-chlorphenyl)-4,4-dimethylpentan-3-on werden in 100 ml wasserfreiem Äther gelöst. Diese Lösung wird zu einer Suspension von 5,7 g Lithiumalanat in 200 ml wasserfreiem Äther langsam hinzugetropft. Das Gemisch wird drei Stunden unter Rückfluß erhitzt und anschließend unter Eiskühlung tropfenweise mit 150 ml Eiswasser versetzt. Danach wird das Gemisch noch mit 200 ml 20 prozentiger (Gewichtsprozent) wäßriger Natronlauge gerührt und mit je 150 ml Äther dreimal extrahiert. Die organische Phase wird über $Na_2SO_4$ getrocknet und im Vakuum eingeengt. Der teilweise kristallisierte Niederschlag wird mit Petroläther verrührt und gewaschen.

Man erhält 24,9 g (85% d. Th.) 1-[1,2,4-Triazolyl-(1)]-1-(4′-chlor-phenyl)-4,4-dimethyl-pentan-3-ol vom Schmelzpunkt 104—106°C.

In entsprechender Weise können die in der folgenden Tabelle 2 aufgeführten Verbindungen hergestellt werden.

TABELLE 2

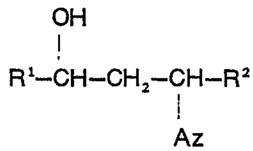

| Nr. | $R^1$ | $R^2$ | Az | Fp °C | IR–Banden $cm^{-1}$ |
| --- | --- | --- | --- | --- | --- |
| 2 | $CH_3$ | $(CH_3)_3C-$ | 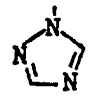 | Harz | |
| 3 | $(CH_3)_2CH-$ | 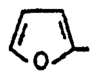 | 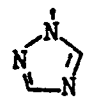 | Harz | 745, 1015, 1140, 1280, 1505, 2970, 3110, 3450 (Film) |
| 4 | $(CH_3)_2CH-$ | 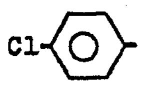 | 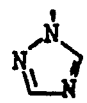 | Harz | 675, 1010, 1088, 1270, 1490, 2875, 2950, 3300 (Film) |
| 5 | sek.-$C_4H_9$ | 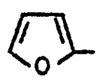 | 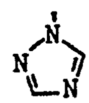 | Harz | 680, 745, 1015, 1135, 1280, 1503, 2890, 3280 (Film) |
| 6 | iso-$C_4H_9$ | 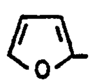 | 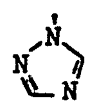 | Harz | 748, 1007, 1135, 1274, 1505, 2920, 2960, 3280 (Film) |
| 7 | $(CH_3)_3C-$ | 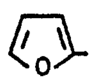 | 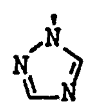 | Harz | 685, 750, 1075, 1280, 1506, 2890, 2970, 3380 (Film) |
| 8 | $(CH_3)_3C-$ | 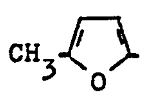 | 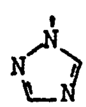 | Harz | 780, 1005, 1133, 1270, 2950, 3110, 3375 (Film) |
| 9 | $(CH_3)_3C-$ | 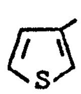 | 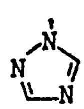 | Harz | 766, 1005, 1075, 1275, 1365, 1500, 2982, 3350 (Film) |
| 10 | sek.-$C_4H_9$– | 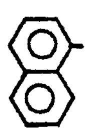 | 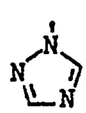 | 97–99 | |

TABELLE 2

| Nr. | $R^1$ | $R^2$ | Az | Fp °C | IR–Banden $cm^{-1}$ |
|---|---|---|---|---|---|
| 11 | iso-$C_4H_9$– | 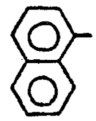 | 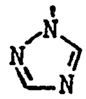 | 94–96<br>105–107 | (Threo-)<br>(Eryhtro-) |
| 12 | $(CH_3)_3C$– | 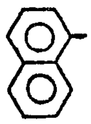 | 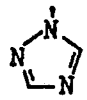 | 100–105 | |
| 13 | $(CH_3)_3C$– | 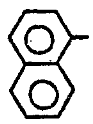 | 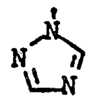 | Harz | 745, 772, 790, 1005, 1390, 2976, 3040, 3400 (Film) |
| 14 | 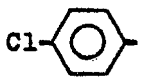 | $(CH_3)_3C$– | 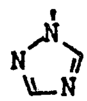 | 83–86 | |
| 15 | 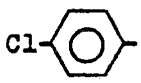 | $(CH_3)_3C$– | 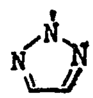 | 68–70 | |
| 16 | $(CH_3)_3C$– | 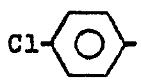 | 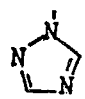 | Harz | 660, 915, 1012, 1075, 1090, 1490, 2955, 3400 (KBr) |
| 17 | $(CH_3)_3C$– | 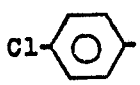 | 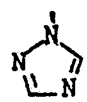 | Harz | 750, 1015, 1090, 1395, 1490, 2875, 2960, 3400 (Film) |
| 18 | $(CH_3)_3C$– | 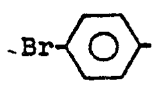 | 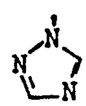 | 120–122 | |
| 19 | $(CH_3)_3C$– | 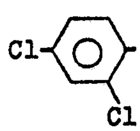 | 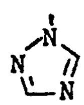 | 118–120 | |
| 20 | $(CH_3)_3C$– | 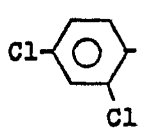 | 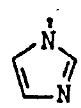 | Harz | 662, 735, 820, 1075, 1470, 1585, 2960, 3360 (KBr) |

TABELLE 2

| Nr. | $R^1$ | $R^2$ | Az | Fp °C | IR–Banden $cm^{-1}$ |
|---|---|---|---|---|---|
| 21 | $(CH_3)_3C–$ | 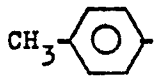 | 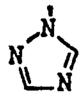 | Harz | 680, 790, 1080, 1135, 1510, 2885, 2960, 3400 (KBr) |
| 22 | $(CH_3)_3C–$ | 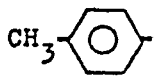 | 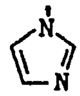 | 98–99 | |
| 23 | $(CH_3)_3C–$ | 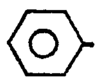 | 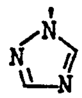 | Harz | 665, 700, 1010, 1140, 1265, 1505, 2885, 2960, 3370 (Film) |
| 24 | $(CH_3)_3C–$ | 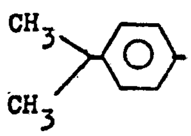 | 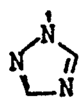 | 102–104 | |
| 25 | $(CH_3)_3C–$ | 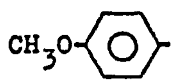 | 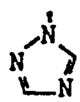 | Harz | 660, 680, 1030, 1250, 1512, 1610, 2950, 3360 (Film) |
| 26 | $(CH_3)_3C–$ | 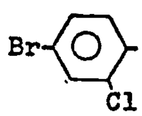 | 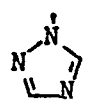 | 154–156 | |
| 27 | $(CH_3)_3C–$ | 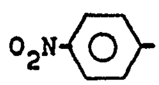 | 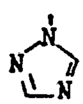 | Harz | 750, 886, 1073, 1137, 1352, 1525, 2980, 3035, 3250 (Film) |
| 28 | 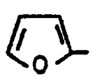 | 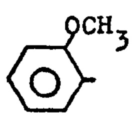 | 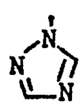 | Harz | 750, 1008, 1131, 1241, 1488, 1596, 2925, 3220 (Film) |
| 29 | 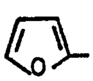 | 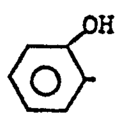 | 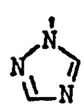 | Harz | 740, 1000, 1125, 1270, 1450, 1497, 1595, 2910, 3230 (Film) |
| 30 | 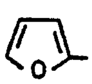 | 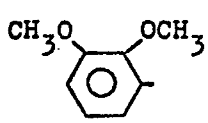 | 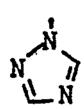 | Harz | 748, 1005, 1060, 1272, 1479, 1585, 2930, 3290 (Film) |

TABELLE 2

| Nr. | $R^1$ | $R^2$ | Az | Fp °C | IR–Banden $cm^{-1}$ |
|---|---|---|---|---|---|
| 31 | 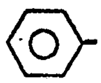 | 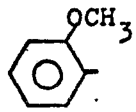 | 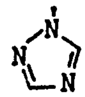 | Harz | 753, 830, 1028, 1245, 1491, 1509, 1601, 1609, 2830, 2930, 2950, 3350 (Film) |
| 32 | 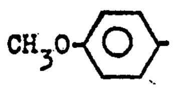 | 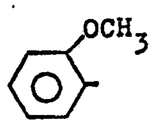 | 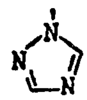 | Harz | 697, 750, 1023, 1240, 1487, 1596, 2925, 3330 (Film) |
| 33 | 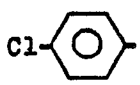 | 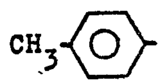 | 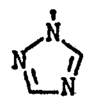 | 137–139 | |
| 34 | 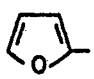 | 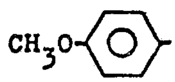 | 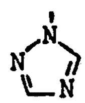 | Harz | 743, 1009, 1032, 1140, 1180, 1252, 1511, 1607, 2833, 2930, 3112, 3270 (Film) |
| 35 | 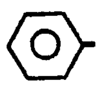 | 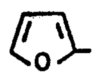 | 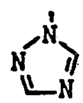 | Harz | 694, 738, 1005, 1130, 1268, 1433, 3330 (Film) |
| 36 | 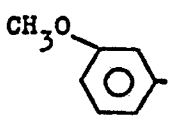 | 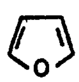 | 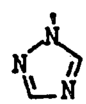 | Harz | 740, 1006, 1035, 1133, 1260, 1480, 1595, 2928, 3320 (Film) |
| 37 | 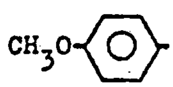 | 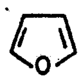 | 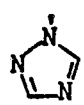 | Harz | 742, 828, 1009, 1030, 1242, 1501, 1607, 2925, 3320 (Film) |
| 38 | $(CH_3)_3C-$ | 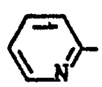 | 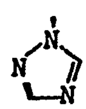 | Harz | 665, 748, 1005, 1072, 1132, 1370, 1428, 1470, 1585, 2950, 3350 (Film) |
| 39 | 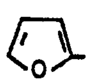 | $(CH_3)_3C-$ | 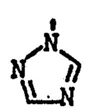 | 113–115 | |

TABELLE 2

| Nr. | $R^1$ | $R^2$ | Az | Fp °C | IR–Banden $cm^{-1}$ |
|---|---|---|---|---|---|
| 40 | 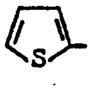 | $(CH_3)_3C-$ | 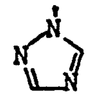 | 148–150 | |
| 41 | 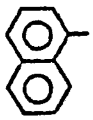 | $(CH_3)_3C-$ | 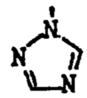 | 178–180 | |
| 42 | 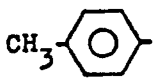 | $(CH_3)_3C-$ | 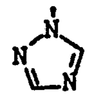 | Harz | 820, 1140, 1275, 1505, 2960, 3020 (Film) |
| 43 | 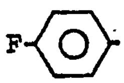 | $(CH_3)_3C-$ | 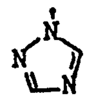 | Harz | 680, 840, 1135, 1220, 1510, 1605, 2960, 3350 (Film) |
| 44 | 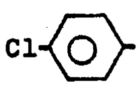 | $(CH_3)_3C-$ | 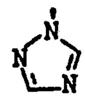 | Harz | 834, 1012, 1090, 1140, 1490, 1510, 2975, 3110, 3300 (Film) |
| 45 | 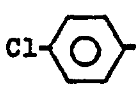 | $(CH_3)_3C-$ | 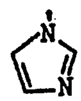 | Harz | 740, 835, 1015, 1090, 1230, 1490, 2970, 3350 (Film) |
| 46 | 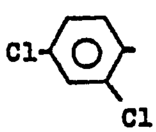 | $(CH_3)_3C-$ | 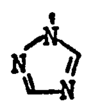 | 130–132 | |
| 47 | 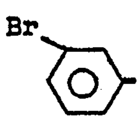 | $(CH_3)_3C-$ | 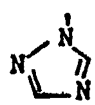 | Harz | 700, 750, 1068, 1140, 1201, 1276, 1370, 1502, 2965, 3300 (Film) |
| 48 | 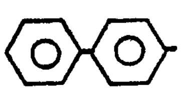 | $(CH_3)_3C-$ | 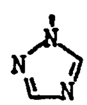 | Harz | 699, 769, 843, 1009, 1139, 1273, 1370, 1486, 1502, 2965, 3320 (Film) |
| 49 | $(CH_3)_3C-$ | $(CH_3)_3C-$ | 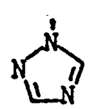 | 120° | |

TABELLE 2

| Nr. | $R^1$ | $R^2$ | Az | Fp °C | IR–Banden $cm^{-1}$ |
|---|---|---|---|---|---|
| 50 | 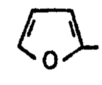 | 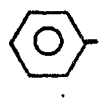 | 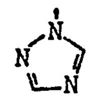 | Harz | 696, 735, 1002, 1131, 1271, 1496, 2912, 3260 (Film) |
| 51 | 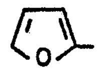 | 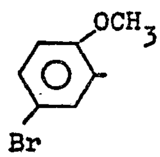 | 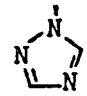 | Harz | 735, 804, 882, 1005, 1019, 1245, 1482, 2930, 3330 (Film) |
| 52 | $(CH_3)_3C-$ | 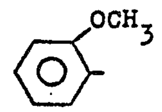 | 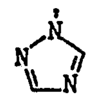 | Harz | 752, 1005, 1243, 1459, 1488, 1598, 2950, 3360 (Film) |
| 53 | 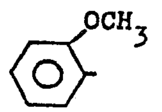 | 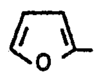 | 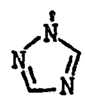 | Harz | |
| 54 | 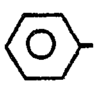 | 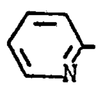 | 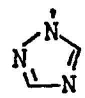 | Harz | |
| 55 | 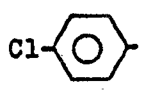 | 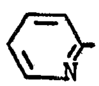 | 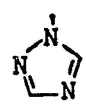 | Harz | 832, 1012, 1080, 1137, 1276, 1433, 1489, 1592, 3300 (Film) |
| 56 | 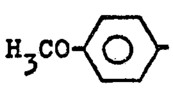 | 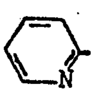 | 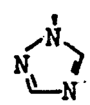 | Harz | 829, 1026, 1167, 1241, 1504, 1590, 330 (Film) |

Die Anwendung der erfindungsgemäßen Wirkstoffe erfolgt z.B. in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsmäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkte, wie Kerosin oder Dieselöl, ferner Kohlenteeröle usw., sowie Öle pflanzlischen oder tierischen Ursprungs, aliphatische, cyclische und aromatische Kohlenwasserstoffe, zum Beispiel Loluol, Xylol, Paraffin, Tetrahydronaphthalin, alkylierte Naphthaline oder deren Derivate, Alkohole (z.B. Methanol, Butanol), Amine (z.B. Äthanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyäthylen-Fettalkohol-Äther, Alkylsulfonate und Arylsulfonate) und Dispergiermittel, wie Lignin, Sulfitablaugen und Methylcellulose.

Die Formulierungen enthalten im allgemeinen zwischen 0,1 und 95 Gewichtsprozent Wirkstoff, vorzugsweise zwischen 0,5 und 90%.

Die erfindungsgemäßen Mittel können in diesen Aufwendungsformen auch zusammen mit anderen Wirkstoffen vorliegen, z.B. Herbiziden, Insektiziden, Wachstumsregulatoren und Fungiziden oder auch mit Düngemittel vermischt werden.

Für die Anwendungszeit gilt, daß die Anwendung der erfindungsgemäßen Wirkstoffe in einem günstigen Zeitraum vorgenommen wird, dessen genaue Feststellung sich nach den geographischen, klimatischen und vegetativen Gegebenheiten richten.

In den folgenden Beispielen wird die Wirkung der erfindungsgemäßen Stoffe als Wachstumsregulatoren dargestellt, ohne die Möglichkeit weiterer Anwendungen als Wachstumsregulatoren auszuschließen.

BEISPIEL 2

Zur Prüfung der biologischen Wirksamkeit der neuen Verbindungen wurden im Gewächshaus Versuche durchgeführt. Dabei wurden die Testpflanzen unter Verwendung von Torfkultursubstrat, welches ausreichend mit Nährstoffen versorgt war, in Kunststoffgefäße mit einem Durchmesser von 12,5 cm eingesät. Die Anwendung der Wirkstoffe erfolgt in wäßriger Lösung oder Dispersion in unterschiedlichen Aufwandmengen auf den Boden am Tage der Einsaat. Die Substanzen wurden dabei direkt auf die Bodenoberfläche gespritzt. Während der Wachstumszeit von 18 Tagen zeigten die mit den erfindungsgemäßen Mitteln behandelten Pflanzen gegenüber der unbehandelten Kontrolle ein deutlich geringeres Längenwachstum, was durch anschliessende Längenmessungen bestätigt werden konnte. Hierbei wurden von jeder Behandlungsreihe 100 Pflanzen gemessen.

Der bekannte Wirkstoff 2-Chloräthyl-trimethyl-ammoniumchlorid (A) zeigte eine schlechtere Wirkung (Ergebnisse s. Tabelle 3—5).

TABELLE 3

Einfluß auf das Längenwachstum von Weizen

| Wirkstoff Nr. | Aufwandmenge kg/ha | Pflanzenhöhe cm | relativ |
|---|---|---|---|
| Kontrolle (unbehandelt) | – | 29,5 | 100 |
| A | 3,0 | 21,1 | 71,5 |
| | 12,0 | 19,3 | 65,4 |
| 7 | 3,0 | 25,5 | 86,4 |
| | 12,0 | 17,0 | 57,6 |

TABELLE 4

Einfluß auf das Längenwachstum von Gerste

| Wirkstoff Nr. | Aufwandmenge kg/ha | Pflanzenhöhe cm | relativ |
|---|---|---|---|
| Kontrolle (unbehandelt) | – | 28,5 | 100 |
| A | 3,0 | 23,3 | 81,8 |
| | 12,0 | 21,5 | 75,4 |
| 19 | 3,0 | 23,5 | 82,5 |
| | 12,0 | 20,5 | 71,9 |
| 1 | 3,0 | 23,5 | 82,5 |
| | 12,0 | 20,0 | 70,2 |

TABELLE 5

Einfluß auf das Wachstum von Raps

| Wirkstoff Nr. | Aufwandmenge kg/ha | Pflanzenhöhe cm | Pflanzenhöhe relative |
|---|---|---|---|
| Kontrolle | – | 16,0 | 100 |
| A | 3,0 | 16,0 | 100 |
| | 12,0 | 16,0 | 100 |
| 7 | 3,0 | 15,0 | 93,8 |
| | 12,0 | 10,5 | 65,6 |
| 1 | 3,0 | 15,0 | 93,8 |
| | 12,0 | 11,0 | 68,8 |
| 19 | 3,0 | 15,0 | 93,8 |
| | 12,0 | 11,5 | 71,9 |
| 23 | 3,0 | 15,0 | 93,8 |
| | 12,0 | 12,0 | 75,0 |
| 21 | 3,0 | 15,0 | 93,8 |
| | 12,0 | 12,0 | 75,0 |

BEISPIEL 3

Man vermischt 90 Gewichtsteile der Verbindung 3 mit 10 Gewichtsteilen N-Methyl-$\alpha$-pyrrolidon und erhält eine Lösung, die zur Anwendung in Form kleinster Tropfen geeignet ist.

BEISPIEL 4

20 Gewichsteile der Verbindung 4 werden in einer Mischung gelöst, die aus 80 Gewichsteilen Xylol, 10 Gewichsteilen des Anlagerungsproduktes von 8 bis 10 Mol Äthylenoxid an 1 Mol Ölsäure-N-mono-äthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

BEISPIEL 5

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

BEISPIEL 6

20 Gewichtsteile der Verbindung 4 werden in einer Mischung gelöst, die aus 25 Gewichtsteilen Cyclohexanol, 65 Gewichtsteilen einer Mineralölfraktion vom Siedepunkt 210 bis 280°C und 10

Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

BEISPIEL 7

20 Gewichtsteile des Wirkstoffs 5 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

BEISPIEL 8

3 Gewichtsteile der Verbindung 3 werden mit 97 Gewichtsteilen feinteiliges Kaolin innig vermischt. Man erhält auf diese Weise ein Stäubemittel, das 3 Gewichtsprozent des Wirkstoffs enthält.

BEISPIEL 9

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

BEISPIEL 10

40 Gewichtsteile des Wirkstoffs 3 werden mit 10 Teilen Natriumsalz eines Phenolsulfonsäureharnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

BEISPIEL 11

20 Teile des Wirkstoffs 4 werden mit 2 Teilen Calciumsalz der Dodecylbenzolsulfonsäure, 8 Teilen Fettalkohol-polyglykoläther, 2 Teile Natriumsalz eines Phenolsulfonsäure-harnstoff-formaldehydkondensats und 68 Teile eines paraffinischen Mineralöls innig vermischt. Man erhält eine stabile ölige Dispersion.

**Patentansprüche**

1. $\gamma$-Azolyl-alkohole der Formel

$$R^1-\underset{\substack{|\\ OH}}{CH}-CH_2-\underset{\substack{|\\ Az}}{CH}-R^2$$

in der $R^1$ und $R^2$ gleich oder verschieden sind und Alkyl mit bis zu 5 C-Atomen, Cycloalkyl, Furanyl, Thiophenyl, Pyridyl, Naphthyl oder Phenyl bedeuten, wobei der Phenylrest durch Halogen, Alkyl, Alkoxy, Alkenyloxy, Hydroxy, Nitro oder Trifluormethyl substituiert sein kann und $R^1$ auch noch Biphenyl sein kann und Az einen Imidazolyl-, Pyrazolyl-, 1,2,4-Triazolyl- oder 1,2,3-Triazolyl-rest bedeutet.

2. 1-[1,2,4-Triazolyl-(1)]-1-[furanyl-(2)]-4,4-dimethylpentan-3-ol.

3. 1-[1,2,4-Triazolyl-(1)]-1-(phenyl)-4,4-dimethylpentan-3-ol.

4. 1-[1,2,4-Triazolyl-(1)]-1-(2′,4′-dichlorphenyl)-4,4-dimethylpentan-3-ol.

5. 1-[1,2,4-Triazolyl-(1)]-1-(4′-chlorphenyl)-4,4-dimethylpentan-3-ol.

6. 1-[1,2,4-Triazolyl-(1)]-1-(1′-naphthyl)-4,4-dimethylpentan-3-ol.

7. 1-[1,2,4-Triazolyl-(1)]-1-(4′-methylphenyl)-4,4-dimethylpentan-3-ol.

8. 1-[1,2,4-Triazolyl-(1)]-1-tertiär-butyl-3-(4′-chlorphenyl)-propan-3-ol.

9. Mittel zur Regulierung des Pflanzenwachstums, enthaltend einen $\gamma$-Azolylalkohol gemäß Anspruch 1.

10. Mittel gemäß Anspruch 9, enthaltend eine Verbindung, ausgewählt aus der Gruppe bestehend aus

1-[1,2,4-Triazolyl-(1)]-1-[Furanyl-(2)]-4,4-dimethylpentan-3-ol
1-[1,2,4-Triazolyl-(1)]-1-(phenyl)-4,4-dimethylpentan-3-ol
1-[1,2,4-Triazolyl-(1)]-1-(2′,4′-dichlorphenyl)-4,4-dimethyl-pentan-3-ol
1-[1,2,4-Triazolyl-(1)]-1-(4′-chlorphenyl)-4,4-dimethylpentan-3-ol
1-[1,2,4-Triazolyl-(1)]-1-(1′-naphthyl)-4,4-dimethylpentan-3-ol
1-[1,2,4-Triazolyl-(1)]-1-(4′-methylphenyl)-4,4-dimethylpentan-3-ol
1-[1,2,4-Triazolyl-(1)]-1-tertiär-butyl-3-(4′-chlorphenyl)-propan-3-ol.

11. Verfahren zur Beeinflussung des Pflanzenwachstums, dadurch gekennzeichnet, daß man die Pflanzen mit einem $\gamma$-Azolylalkohol gemäß Anspruch 1 behandelt.

## Claims

1. A $\gamma$-azolyl alcohol of the formula

$$R^1—CH—CH_2—CH—R^2$$
$$\quad\; | \qquad\qquad\;\; |$$
$$\quad OH \qquad\quad Az$$

where $R^1$ and $R^2$ are identical or different and each denotes alkyl of a maximum of 5 carbon atoms, cycloalkyl, furanyl, thiophenyl, pyridyl, naphthyl, unsubstituted phenyl, or phenyl substituted by halogen, alkyl, alkoxy, alkenyloxy, hydroxy, nitro or trifluoromethyl, and $R^1$ may also be biphenyl, and Az denotes imidazolyl, pyrazolyl, 1,2,4-triazolyl or 1,2,3-triazolyl.

2. 1-[1,2,4-Triazolyl-(1)]-1-[furanyl-(2)]-4,4-dimethylpentan-3-ol.
3. 1-[1,2,4-Triazolyl-(1)]-1-(phenyl)-4,4-dimethylpentan-3-ol.
4. 1-[1,2,4-Triazolyl-(1)]-1-(2',4'-dichlorophenyl)-4,4-dimethylpentan-3-ol.
5. 1-[1,2,4-Triazolyl-(1)]-1-(4'-chlorophenyl)-4,4-dimethylpentan-3-ol.
6. 1-[1,2,4-Triazolyl-(1)]-1-(1'-naphthyl)-4,4-dimethylpentan-3-ol.
7. 1-[1,2,4-Triazolyl-(1)]-1-(4'-methylphenyl)-4,4-dimethylpentan-3-ol.
8. 1-[1,2,4-Triazolyl-(1)]-1-tert-butyl-3-(4'-chlorophenyl)-propan-3-ol.
9. An agent for influencing the growth of plants, containing a $\gamma$-azolyl alcohol according to claim 1.
10. An agent according to claim 9, containing a compound selected from the group consisting of
1-[1,2,4-triazolyl-(1)]-1-[furanyl-(2)]-4,4-dimethylpentan-3-ol
1-[1,2,4-triazolyl-(1)]-1-(phenyl)-4,4-dimethylpentan-3-ol
1-[1,2,4-triazolyl-(1)]-1-(2',4'-dichlorophenyl)-4,4-dimethylpentan-3-ol
1-[1,2,4-triazolyl-(1)]-1-(4'-chlorophenyl)-4,4-dimethylpentan-3-ol
1-[1,2,4-triazolyl-(1)]-1-(1'-naphthyl)-4,4-dimethylpentan-3-ol
1-[1,2,4-triazolyl-(1)]-1-(4'-methylphenyl)-4,4-dimethylpentan-3-ol
1-[1,2,4-triazolyl-(1)]-1-tert-butyl-3-(4'-chlorophenyl)-propan-3-ol.
11. A process for influencing the growth of plants, characterized in that the plants are treated with a $\gamma$-azolyl alcohol according to claim 1.

## Revendications

1. Alcohol $\gamma$-azolylique de la formule

$$R^1—CH—CH_2—CH—R^2$$
$$\quad\; | \qquad\qquad\;\; |$$
$$\quad OH \qquad\quad Az$$

dans laquelle $R^1$ et $R^2$ sont identiques ou différents et représentent un groupe alkyle en $C_1—C_5$, cycloalkyle, furanyle, thiophényle, pyridyle, naphtyle ou phényle, le groupe phényle pouvant être substitué par un halogène, un groupe alkyle, alcoxy, alcényloxy, hydroxy, nitro ou trifluorométhyle et $R^1$ peut être aussi un groupe biphényle, et Az représente un groupe imidazolyle, pyrazolyle, 1,2,4-triazolyle ou 1,2,3-triazolyle.

2. 1-[1,2,4-triazolyl-(1)]-1-[furanyl-(2)]-4,4-diméthylpentane-3-ol.
3. 1-[1,2,4-triazolyl-(1)]-1-(phényl)-4,4-diméthylpentane-3-ol.
4. 1-[1,2,4-triazolyl-(1)]-1-(2',4'-dichlorophényl)-4,4-diméthylpentane-3-ol.
5. 1-[1,2,4-triazolyl-(1)]-1-(4'-chlorophényl)-4,4-diméthylpentane-3-ol.
6. 1-[1,2,4-triazolyl-(1)]-1-(1'-naphthyl)-4,4-diméthylpentane-3-ol.
7. 1-[1,2,4-triazolyl-(1)]-1-(4'-méthylphényl)-4,4-diméthylpentane-3-ol.
8. 1-[1,2,4-triazolyl-(1)]-1-ter-butyl-3-(4'-chlorophényl)-propane-3-ol.
9. Agent pour la régulation de la croissance des plantes, contenant un alcool $\gamma$-azolylique selon la revendication 1.
10. Agent selon la revendication 9, contenant un composé choisi dans le groupe comprenant.
le 1-[1,2,4-triazolyl-(1)]-1-[furanyl-(2)]-4,4-diméthylpentane-3-ol
le 1-[1,2,4-triazolyl-(1)]-1-(phényl)-4,4-diméthylpentane-3-ol
le 1-[1,2,4-triazolyl-(1)]-1-(2'.4'-dichlorophényl)-4,4-diméthyl-pentane-3-ol
le 1-[1,2,4-triazolyl-(1)]-1-(4'-chlorophényl)-4,4-diméthylpentane-3-ol
le 1-[1,2,4-triazolyl-(1)]-1-(1'-naphthyl)-4,4-diméthylpentane-3-ol
le 1-[1,2,4-triazolyl-(1)]-1-(4'-méthylphényl)-4,4-diméthylpentane-3-ol
le 1-[1,2,4-triazolyl-(1)]-1-tert-butyl-3-(4'-chlorophényl)-propane-3-ol.
11. Procédé pour agir sur la croissance des plantes, caractérisé par le fait qu'on traite les plantes avec un alcool $\gamma$-azolylique selon la revendication 1.